Europäisches Patentamt

(19) European Patent Office

Office européen des brevets

(11) Publication number: **0 173 529**
**B1**

(12) **EUROPEAN PATENT SPECIFICATION**

(45) Date of publication of patent specification: **20.06.90**

(21) Application number: **85305878.2**

(22) Date of filing: **19.08.85**

(51) Int. Cl.⁵: **C 12 N 15/00,** C 12 N 7/00,
A 61 K 39/21

(54) **Molecular clones of the genome of HTLV-III.**

(30) Priority: **22.08.84 US 643306**

(43) Date of publication of application:
**05.03.86 Bulletin 86/10**

(45) Publication of the grant of the patent:
**20.06.90 Bulletin 90/25**

(84) Designated Contracting States:
**AT BE CH DE FR GB IT LI LU NL SE**

(56) References cited:
**EP-A-0 113 078**
**EP-A-0 230 784**
**WO-A-85/04897**

**SCIENCE vol. 224. 4.5.1984, pp. 497-500**
**SCIENCE vol. 224, 4.5.1984, pp. 500-503**
**Proceedings of the National Academy of**
**Sciences of the United States of America, vol.**
**79, no. 22, November 1982, Baltimore, USA M.**
**SEIKI et al. "Human adult T-cell leukemia virus:**
**Molecular cloning of the provirus DNA and the**
**unique terminal structure" pages 6899-6902.**

(73) Proprietor: **THE UNITED STATES OF AMERICA**
**as represented by the Secretary U.S.**
**Department of Commerce**
**National Technical Information Service Office of**
**Government Inventions and Patents 5285 Port**
**Royal Road**
**Springfield, Virginia 22161 (US)**

(72) Inventor: **Wong-Staal, Flossie**
**8418 Harker Dr.**
**Potomac Maryland 20854 (US)**
Inventor: **Gallo, Robert C.**
**8513 Thornden Terrace**
**Bethesda Maryland 20817 (US)**
Inventor: **Hahn, Beatrice H.**
**3123 Brooklawn Terrace**
**Chevy Chase Maryland 20815 (US)**
Inventor: **Popovic, Mikulas**
**3 Pooks Hill Road 401**
**Bethesda Maryland 20814 (US)**

(74) Representative: **Daley, Michael John et al**
**F.J. CLEVELAND & COMPANY 40/43 Chancery**
**Lane**
**London, WC2A 1JQ (GB)**

Courier Press, Leamington Spa, England.

## Description

In related inventions HTLV-III was detected, isolated and immortalized in an HT cell line (see WO—A—85/04897). Since evidence now strongly indicates that HTLV-III is related to acquired immune deficiency syndrome (AIDS), the ability to enhance production of the virus and determine the DNA sequences of the virus is critically important to developing a cure or reagent active against AIDS, Science Vol. 224, 4.5.84, pp. 497—500 and 500—503. The present invention takes one such significant step by disclosing the process for production of molecular clones of the HTLV-III from a fraction enriched from the unintegrated provirus in acutely infected cells such as the cell line designated H9/HTLV-III. Two forms of this virus are identified which are highly related but differ in several restriction enzyme cleavage sites. Both variants exist as integrated and unintegrated forms in the infected cell line. The complete genomes of two forms of HTLV-III are molecularly cloned and shown to exist in the long-term infected cell line both as polyclonally integrated provirus and as unintegrated viral DNA. These clones are used as probes to detect viral sequences in cell lines other than H9/HTLV-III, taken from different AIDS patients, and in freh lymphoid tissues of AIDS patients, providing further evidence that the cloned genomes constitute predominant forms of HTLV-III, the causative agent in AIDS.

Previous work with the HTLV family of virus showed three variants. Of these, it was believed that HTLV-III was the causative agent of AIDS. Using the clones produced by this invention HTLV-III has been shown to be distinctly different than HTLV-I and HTLV-II, whereas HTLV-I and -II share greater homology and thus better identification of AIDS virus in sera.

Description of the drawings

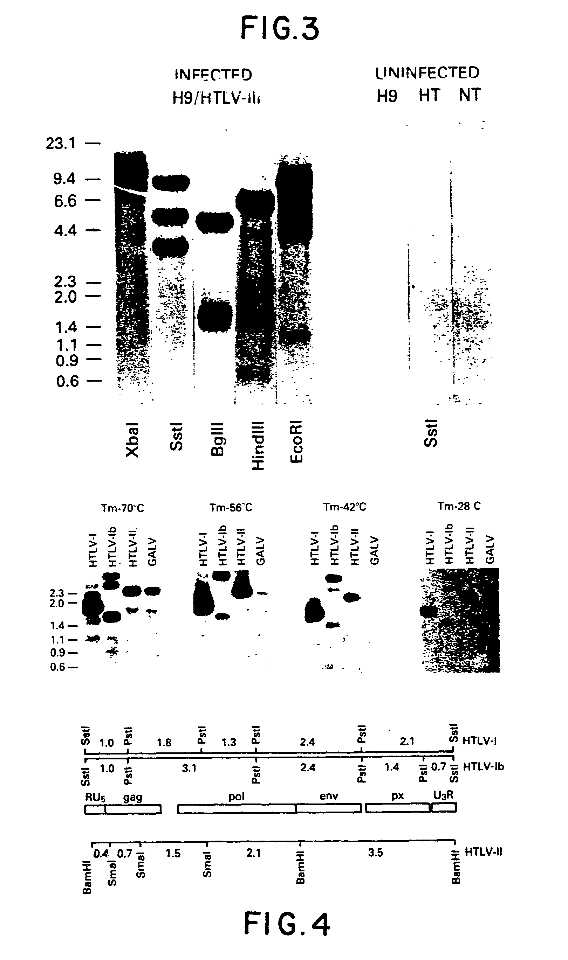

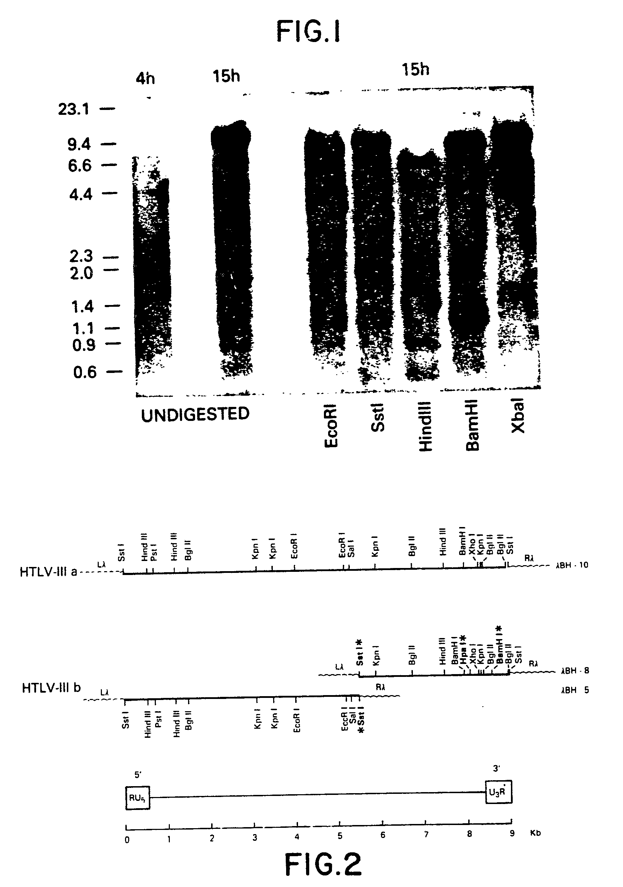

Figure 1 is a Southern blot analysis of unintegrated DNA of HTLV-III. No viral sequences could be detected in the undigested DNA after 4 hours. However, a major species of viral DNA of approximately 10 Kb length was present in the 10, 15, 24 and 48 hr harvest representing the linear unintegrated form of the virus. A representative Southern blot of the 15 hr harvest digested with several restriction enzymes is shown in this figure. Methods: $8 \times 10^8$ fresh uninfected H9 cells were infected with concentrated supernatant from cell line H9/HTLV-III containing $4 \times 10^{11}$ particles of HTLV-III. Infected cells were divided into five Roller bottles and harvested after 4, 10, 15, 24 and 48 hrs. Low molecular weight DNA was prepared using the Hirt fractionation procedure and 30 μg of undigested and digested DNA were separated on a 0.8% agarose gel, transferred to nitrocellulose paper and hybridized to a HTLV-III cDNA probe for 24 hr at 37°C in 1×SSC, 40% formamide and 10% Dextran sulfate. cDNA was synthesized from poly(A) selected RNA prepared from doubly banded HTLV-III virus in the presence of oligo(dT) primers. Filters were washed at 1×SSC at 65°C.

Figure 2 is a restriction endonuclease map of two closely related HTLV-III variants cloned from unintegrated viral DNA. Three recombinant clones (λBH10, λBH5 and λBH8) were analyzed and their inserts (9 Kb, 5.5 Kb and 3.5 Kb, respectively) were mapped with the indicated enzymes. They represent two variant forms of HTLV-III differing in three enzyme sites which are depicted in bold letters and by an asterisk. As SstI cuts the LTR of the HTLV-III the three clones represent two full length genomes with one LTR. A schematic map of this viral genome is shown at the bottom of the figure, although the total length of the LTR is approximate. Methods: Low molecular weight DNA combined from the 15 and 24 hr harvest was fractionated on a 10—40% sucrose gradient. Aliquots of the fractions were electrophoresed on a 0.5% agarose gel, transferred to nitrocellulose paper and hybridized to HTLV cDNA under conditions described in Figure 1. Fractions which contained the unintegrated linear HTLV-III genome shown by hybridization were pooled, the DNA was subsequently digested with SstI and ligated to phosphatase treated SstI arms of λgtWes λB. After *in vitro* packaging, recombinant phages were screened for viral sequences with HTLV-III cDNA.

Figure 3 demonstrates HTLV-III viral sequences in the infected cell line H9/HTLV-III. Both variant forms of HTLV-III were detected as integrated provirus as well as unintegrated viral DNA in the infected cell line. However, no viral sequences were found in uninfected H9 cells, uninfected HT cells nor in normal human thymus (NT). Methods: 10 μg of high molecular weight DNA were digested with restriction enzymes as indicated and hybridized to nick translated phage insert from BH10 under the same conditions as described in Figure 1.

Figure 4 shows a sequence homology of HTLV-III to other members of the HTLV family. A schematic restriction map of HTLV-I, HTLV-Ib and HTLV-II is drawn below indicating the length and the location of the generated fragments in respect to the corresponding genomic regions. LTR, *gag, pol, env* and pX regions are drawn to scale according to the published nucleotide sequence of HTLV-I. The bands which are most highly conserved as stringency increases correspond to the *gag/pol* junction region of HTLV-I (1.8 Kb PstI fragment) and HTLV-IIb (3.1 Kb PstI fragment) and to the 3' part of the *pol* region of HTLV-II (2.1 Kb SmaI/BamHI fragment) which do not overlap assuming the same genomic organization in HTLV-II. Fragments corresponding to pX of HTLV-I (2.1 Kb SstI Pst fragment) and HTLV-Ib (1.4 Kb Pst fragment) are less conserved but still visible at Tm −28°C on the original autoradiogram. Digestion of GaLV generates six fragments, none of which show hybridization under medium or high stringency. Methods: Subclones of full length genomes of a prototype HTLV-I, HTLV-Ib, HTLV-III and GaLV (Seato strain) were digested with the

following enzymes, PstI plus SstI (HTLV-I and HTLV-Ib), BamHI plus SmaI (HTLV-II) and Hind III plus SmaI plus XhoI (GaLV). Four replicate filters were prepared and hybridized for 36 hr under low stringency (8×SSC, 20% formamide, 10% Dextran sulfate at 37°C) to nick translated insert of λBH10. Filters were then washed in 1×SSC at different temperatures, 22°C (Tm−70°C) filter 1, 37°C (Tm−56°C) filter 2, 50°C (Tm−42°C) filter 3 and 65°C (Tm−28°C).

The invention

The present invention discloses a method for production of molecular clones of HTLV-III from a fraction enriched from the unintegrated provirus in acutely infected cells. Three clones for the HTLV-III genome were produced using recombinant DNA techniques by isolation and characterising unintegrated viral DNA, cleaving this DNA with the appropriate restriction enzyme, and constructing a phage library capable of being screened by viral cDNA. This process led to the production of three clones: λBH10, containing a 9.0 Kb SstI-Bgl II viral insert; clone BH8 containing a 3.5 Kb SstI-BglIII insert; and clone BH5 containing a 5.5 Kb SstI-SstI viral insert. See Figure 2 for a pictorial representation of the differences between these three clones.

In general, cloning the HTLV-III genome involved isolating unintegrated viral DNA after infection of H9-cells with concentrated HTLV-III virus and cloning this DNA in a lambda phage library to be screened with viral cDNA. The cell line H9/HTLV-III (Vide WO85/04897) produces large quantities of HTLV-III virus and serves as the principal producer cell line for immunological assays used to detect virus specific antigens and antibodies in AIDS sera. Cultures of H9/HTLV-III cells (infected cells) are grown and harvested, followed by extraction of low molecular weight DNA from the newly infected cells. This produces unintegrated viral DNA. The cDNA library is formed using HTLV-III cDNA. This cDNA is then used as a probe for assaying the unintegrated viral DNA. Unintegrated linear DNA (provirus DNA) is then obtained, containing the entire HTLV-III genome, i.e., replication competent. This DNA is then digested in plasmid lambda gt Wes ° lambda B to form clone lambda BH10. The other clones are produced by digesting provirus DNA that does not contain the entire HTLV-III genome.

Two elements of the above process are recombinant DNA procedures, such as, the DNA library and a cDNA probe. The library is formed by taking the total DNA from H9/HTLV-III cells, cutting the DNA into fragments with a suitable restriction enzyme, hybridizing to the fragments to a radiolabeled cDNA probe, joining the fragments to plasmid vectors, and then introducing the recombinant DNA into a suitable host.

The cDNA probe is an HTLV-III cDNA probe made from double-banded HTLV-III mRNA. A short oligo-dT chain is hybridized to the poly-A tail of the mRNA strand. The oligo-T segment serves as a primer for the action of reverse transcriptase, which uses the mRNA as a template for the synthesis of a complementary DNA strand. The resulting cDNA ends in a hairpin loop. Once the mRNA strand is degraded by treatment with NaOH, the hairpin loop becomes a primer for DNA polymerase I, which completes the paired DNA strand. The loop is then cleaved by S1 nuclease to produce a double-stranded cDNA molecule. Linkers are then added to the double-stranded cDNA by using DNA ligase. After the linkers are cut open with a restriction enzyme and the cDNA is inserted into a suitable plasmid cleaved with the same enzyme, such as pBR322, the result is a cDNA-containing recombinant plasmid.

Statement of deposit

The cell lines and clones of this invention are on deposit in the American Type Culture Collection in the manner prescribed by the Patent and Trademark Office with regard to permanence of the deposit for the life of the patent and without restriction on public access. The accession numbers are: H9/HTLV-III, CRL 8543; BH10, #40125; BH8, #40127; and BH5, #40126.

Specific disclosure

Concentrated virus from H9/HTLV-III is used to infect fresh uninfected H9 cells at a multiplicity of 50 viral particles/cell; cultures are harvested after 4, 10, 15, 24 and 48 hours. Extrachromosomal DNA is extracted according to the procedure of Hirt (J. Molec. Biol. Vol. 26, 1967, pp. 365—369) and assayed for its content of unintegrated viral DNA using HTLV-III cDNA as a probe. This cDNA is primed by oligo(dT) and copied from poly(A) containing RNA from virions that had been twice banded on sucrose density gradients. Unintegrated linear viral DNA is first detected after 10 hrs and is also present at the subsequent time points. A Southern blot of the 15 hr harvest is shown in Figure 1. A band of approximately 10 Kb in the undigested DNA represents the linear form of the unintegrated, replication-competent HTLV-III. No closed or nicked circular DNA could be detected in the 10, 15 and 24 hour harvest, but both forms were evident in small amounts at the 48 hr harvest (data not shown). The viral genome was not cut by XbaI, whereas SstI generated three predominant bands of 9 Kb, 5.5 Kb and 3.5 Kb (Figure 1). These bands represent the complete genomes of two forms of HTLV-III, both cut by SstI in the LTR and one having an additional SstI site in the middle of its genome. Clone BH10 contains a viral insert of 9.0 Kb, a size consistent with the complete HTLV-III genome. Clones BH8 and BH5 contain inserts of 5.5 Kb and 3.5 Kb, respectively, and together they overlap completely with BH10, except for a few restriction enzyme sites polymorphisms in BH5. Therefore, BH10 and BH8 plus BH5 represents two variants of HTLV-III.

Example 1

In order to demonstrate the presence of these two variants in the original cell line, nick-translated inserts of lambda BH10 was hybridized to a Southern blot of H9/HTLV-III genomic cDNA digested with several restriction enzymes (Figure 3). Both forms could be detected using the enzyme SstI generating the expected 3 bands of 9.0 Kb, 5.5 Kb and 3.5 Kb XbaI which does not cut the provirus generating a high molecular weight genome representing polyclonal integration of the provirus and a band of approximately 10 Kb which could be interpreted as representing unintegrated viral DNA since a band of identical size was also present in the undigested first preparation (Figure 1). This was confirmed by Southern blot hybridization of undigested cellular DNA. The existence of unintegrated viral DNA thus explains the presence of a 4 Kb and 4.5 Kb EcoRI fragment seen in both first and total cellular DNA preparations (Figure 1 and Figure 3). BglII and HindIII both cut the LTR and generated the expected internal bands. Several faint bands in the HindIII digest, in addition to the internal bands, represent either defective proviruses or another variant form with differences in the HindIII restriction pattern. The lack of HTLV-III sequences in the uninfected H9 cell line and the uninfected parental line HT as well as in normal human thymus demonstrated the exogenous nature of HTLV-III and showed that the virus does not contain any human cellular sequences. The same results were obtained using nick-translated inserts from lambda BH5 and lambda BH8.

Example 2

The availability of the cloned HTLV-III genome allowed sequence homology between HTLV-III, HTLV-I and HTLV-II to be evaluated. Replicate Southern blots of restriction enzyme digested clones representing the complete genomes of HTLV-I, HTLV-Ib, HTLV-II and GALV as a control were hybridized to full length HTLV-III probe under relaxed conditions. The filters were then washed (λBH10i) under conditions of low, medium and high stringencies in order to estimate the extent of homology between HTLV-III and these viruses (Figure 4). This experiment showed that there is specific homology between HTLV-III, HTLV-I, HTLV-Ib and HTLV-II but not with HTLV-III and GALV. As demonstrated, hybridization of HTLV-III to other members of the HTLV family could be detected at the values of −42°C and −28°C, conditions under which no hybridization to GALV was seen (Figure 4, panels C and D). Of note, the restriction fragments showing greatest homology correspond to the *gag/pol* region of HTLV-I and to an apparently non-overlapping portion of the *pol* region of HTLV-II (assuming that the genomic arrangement is similar to that of HTLV-I). Further analysis revealed that it is the 5' half of the *gag* and the cap between *gag* and *pol* which has the greatest homology in HTLV-I. Finally, in HTLV-Ib (a variant of HTLV-I) hybridization to the px region could be seen (1.4 Kb Pst fragment) as well as to the corresponding px fragment in HTLV-I (2.1 Kb Pst/Sst) on the original autoradiogram.

Example 3

Figure 2 shows the restriction map of three clones designated λBH10, λBH5 and λBH8 which correspond in size to the three SstI fragments shown in Figure 1. Comparison of these maps suggest that λBH5 plus λBH8 constitute one HTLV-III genome, and λBH10 another. The two viral forms differ in only three out of 21 mapped enzyme sites, including the internal SstI site. As expected, the phage inserts of λBH5 and λBH8 hybridize under high stringency conditions to λBH10 but not to each other as analyzed by Southern blot hybridization and electron microscopic heteroduplex analysis. To show the presence of LTR sequences in the clones and to determine their orientation, a cDNA clone (C15) was used as a probe and contained U3 and R sequences. This clone strongly hybridized to the 0.5 Kb BglII fragment of λBH10 and λBH8, orienting this side 3', and faintly hybridized to the 0.7 Kb SstI/PstI fragment of λBH5 and λBH10, orienting this side 5', and demonstrated that SstI cuts the LTR of HTLV-III in the R region.

Example 4

The presence of two variant forms of HTLV-III in the original cell line was demonstrated by hybridizing the radiolabelled insert of λBH10 to a Southern blot of H9/HTLV-III genomic DNA digested with several restriction enzymes (Figure 3). Both forms were detected using the enzyme SstI which generated the expected 3 bands of 9 Kb, 5.5 Kb and 3.5 Kb length. Both of these forms are also present as integrated proviruses because they have been cloned along with their flanking cellular sequences from a genomic library of H9/HTLV-III. Furthermore, XbaI, which does not cut the provirus, generated a high molecular weight smear representing polyclonal integration of the provirus and a band of approximately 10 Kb, representing unintegrated viral DNA. This same 10 Kb band was also detected in undigested H9/HTLV-III DNA, again indicating unintegrated viral DNA. The presence of unintegrated viral DNA also explains the 4 Kb and 4.5 Kb EcoRI fragment seen in both the Hirt and total cellular DNA preparations (Figures 1, 3). BglII and HindIII both cut the LTR and generate the expected internal bands. Several faint bands, in addition to the internal bands using HindIII, represent either defective proviruses or another variant form present in low copy number. The lack of HTLV-III sequences in the DNA of the uninfected H9 cell line and the uninfected parental cell line HT as well as in normal human thymus clearly demonstrates the exogenous nature of HTLV-III and shows that the virus does not contain human cellular sequences. The same results were obtained using λBH5 and λBH8 as probe inserts.

Claims

1. The 9 Kb SstI-BglII viral insert obtainable from ATCC 40125, and having the restriction map depicted in Figure 2.

2. The 3.5 Kb SstI-BglII viral insert obtainable

from ATCC 40126, and having the restriction map depicted in Figure 2.

3. The 5.5 Kb SstI-SstI viral insert obtainable from ATCC 40127, and having the restriction map depicted in Figure 2.

4. A process for the production of recombinant clones of HTLV-III which comprises, a) cleaving unintegrated viral DNA from HTLV-III infected cells with a restriction enzyme, b) ligating said DNA into a suitable cloning vector to produce the recombinant clone, and c) hybridizing said recombinant clone to the HTLV-III specific probe of any one of claims 1 to 3 to obtain the recombinant clone.

5. A process for cloning cDNA specific to HTLV-III, characterised by

isolating total cellular mRNA from HTLV-III infected cells;

forming double-stranded cDNA from said mRNA and inserting said double-stranded cDNA into a suitable phage or plasmid vector to form a recombinant DNA molecule;

hybridizing said recombinant DNA molecule with the HTLV-III radiolabelled probe of any one of claims 1 to 3;

removing cDNA from said molecule and inserting said cDNA into a suitable vector; and

transfecting said vector into a suitable host cell.

6. The process of claim 5 wherein said vector is characterised in that it contains a viral insert of any one of claims 1 to 3.

7. A recombinant clone of HTLV-III plasmid λ gt Wes° λB selected from the group held in deposit in the American Type Culture Collection under accession numbers 40125; 40126; and 40127.

8. A pharmaceutical preparation characterised by including a recombinant clone as claimed in claim 7.

9. The use of a recombinant clone as claimed in claim 7 in the preparation of a medicament for the treatment, or diagnosis of acquired immune deficiency syndrome (AIDS).

**Patentansprüche**

1. Der aus ATCC 40125 erhältliche 9-Kb-SstI-BglII-Virusein-Insert mit der Restriktionskarte nach Fig. 2.

2. Der aus ATCC 40126 erhältliche 3.5-Kb-SstI-BglII-Virus-Insert mit der Restriktionskarte nach Fig. 2.

3. Der aus ATCC 40127 erhältliche 5.5-Kb-SstI-BglII-Virus-Insert mit der Restriktionskarte nach Fig. 2.

4. Verfahren zur Herstellung recombinanter HTLV-III-Klone, das umfaßt:

a) Aufspalten nicht integrierter viraler DNS aus mit HTLV-III infizierten Zellen mit einem Restriktionsenzym,

b) Ligieren der DNS in einen geeigneten Konierungsvektor zur Erzeugung des rekombinanten Klons, und

c) Hybridisieren dieses rekombinanten Klons mit der HTLV-III-spezifischen Sonde nach einem der Ansprüche 1 bis 3 zur Gewinnung des rekombinanten Klons.

5. Verfahren zum Klonieren HTLV-III-spezifischer Komplementär-DNS gekennzeichnet durch:

Isolieren vollständiger zellularer Boten-RNS aus HTLV-III-infizierten Zellen,

Herstellen zweisträngiger Komplementär-DNS aus der Boten-RNS und Einführen der zweisträngigen Komplementär-DNS in einen geeigneten Phagen- oder Plasmidvektor zur Bildung eines rekombinanten DNS-Moleküls,

Hybridisieren dieses rekombinanten DNS-Moleküls mit der HTLV-III-radiomarkierten Sonde nach einem der Ansprüche 1 bis 3, Entfernen von Komplementär-DNS aus dem genannten Molekül und Einsetzen der Komplementär-DNS in einen geeigneten Vektor, und

Übertragen dieses Vektors in eine geeignete Wirtszelle.

6. Verfahren nach Anspruch 5, wobei der gennante Vektor dadurch gekennzeichnet ist, daß er einen viralen Insert nach einem der Ansprüche 1 bis 3 enthält.

7. Rekombinantes Klon von HTLV-III-Plasmid-λ gt Wes° λB, das aus der unter den Zugriffsnummern 40125, 40126 und 40127 in der American Type Culture Collection hinterlegten Gruppe ausgewählt ist.

8. Pharmazeutisches Präparat, dadurch gekennzeichnet, daß es ein rekombinantes Klon nach Anspruch 7 enthält.

9. Verwendung eines rekombinanten Klons nach Anspruch 7 bei der Zubereitung eines Medikaments zur Behandlung oder Diagnose des erworbenen Immundefektsyndroms (AIDS).

**Revendications**

1. Insert viral SstI-BglII de 9 Kb pouvant être obtenu à partir d'ATCC 40125 et ayant la carte de restriction illustrée par la figure 2.

2. Insert viral SstI-BglII de 3,5 Kb pouvant être obtenu à partir d'ATCC 40126 et ayant la carte de restriction illustrée par la figure 2.

3. Insert viral SstI-SstI de 5,5 Kb pouvant être obtenu à partir d'ATCC 40127 et ayant la carte de restriction illustrée par la figure 2.

4. Procédé pour la production de clones recombinants de HTLV-III qui comprend a) le clivage d'ADN viral non intégré de cellules infectées par HTLV-III avec une enzyme de restriction, b) la liaison dudit ADN dans un vecteur de clonage approprié pour produire le clone recombinant et c) l'hybridation dudit clone recombinant à la sonde spécifique HTLV-III de l'une quelconque des revendications 1 à 3 pour obtenir le clone recombinant.

5. Procédé pour cloner de l'ADNc spécifique d'HTLV-III, caractérisé par le fait que:

l'on isole l'ARNm cellulaire total de cellules infectées par l'HTLV-III;

l'on forme un ADNc double brin à partir dudit ARNm et l'on insère ledit ADNc double brin dans un vecteur phagique ou plasmidique approprié pour former une molécule d'ADN recombinant;

l'on hybride ladite molécule d'ADN recombinant avec la sonde d'HTLV-III de l'une quelconque des revendications 1 à 3 radiomarquée;

l'on sépare l'ADNc de ladite molécule et l'on insère ledit ADNc dans un vecteur approprié; et

l'on réalise la transfection dudit vecteur dans une cellule hôte appropriée.

6. Procédé selon la revendication 5, dans lequel ledit vecteur est caractérisé en ce qu'il contient un insert viral de l'une quelconque des revendications 1 à 3.

7. Clone recombinant d'HTLV-III plasmide λ gt Wes° λB choisi dans le groupe déposé à l'American Type Culture Collection sous les numéros d'ordre 40125; 40126; et 40127.

8. Préparation pharmaceutique caractérisé en ce qu'elle comprend un clone recombinant selon la revendication 7.

9. Utilisation d'un clone recombinant selon la revendication 7 dans la préparation d'un médicament pour le traitement ou dans le diagnostic du syndrome d'immunodéficience acquise (SIDA).

EP 0 173 529 B1

FIG.3

FIG.4

## FIG.I

23.1 —
9.4 —
6.6 —
4.4 —

2.3 —
2.0 —

1.4 —

1.1 —
0.9 —

0.6 —

4h     15h        15h

UNDIGESTED     EcoRI    SstI    HindIII    BamHI    XbaI

## FIG.2